# DEMANDE DE BREVET EUROPEEN

(11) **EP 3 603 508 A1**
(43) Date de publication de la demande: **05.02.2020**
(21) Numéro de dépôt: 18306065.6
(22) Date de dépôt: 03.08.2018
(51) Int. Cl.: A61B 5/145, A61B 5/00

(54) **CAPSULE MULTI-POSITION**

(71) Demandeur: PKvitality, 75001 Paris (FR)
(72) Inventeur: PIERART, Luc, 94800 VILLEJUIF (FR)
(74) Mandataire: Regimbeau

(57) **Abrégé**

L'invention propose une capsule (220), la capsule (220) comprenant :
- une face de couplage (222) configurée pour être couplée avec une forme complémentaire d'un boitier (120),
- un réseau de microaiguilles (210) positionnées sur une surface plane de contact (229),
dans lequel :
- la face de couplage (222) présente une symétrie de rotation autour d'un axe de rotation (Z) orthogonal à la surface plane (229), autour duquel au moins deux positions de couplage sont autorisées avec une seule et unique forme complémentaire d'un boitier (120),
- aucune micro-aiguille (210) n'est au même emplacement, dans un référentiel fixe, qu'une autre microaiguille (210) dans les deux positions.

## Description

### DOMAINE TECHNIQUE GENERAL

La présente invention se rapporte à un système de surveillance corporelle via analyse de liquide corporel, typiquement interstitiel, à l'aide de microaiguilles.

Plus précisément, la présente invention concerne une capsule comportant des microaiguilles, pour la gestion des microaiguilles dans la peau.

### ETAT DE L'ART

Certaines pathologies comme le diabète nécessitent une surveillance quotidienne de paramètres biochimiques du corps humain, i.e. des concentrations en certains composés (la glycémie dans l'exemple du glucose).

Pour cela, il est courant de piquer un point de la peau de sorte à faire perler une goutte de sang, et d'analyser cette goutte soit de façon réactive (par exemple avec une bandelette), soit de façon électronique (par exemple par au moins d'un capteur analytique), de façon à estimer le ou les paramètres cible.

On connait aujourd'hui des systèmes évolués bien moins invasifs qui se contentent d'analyser le liquide interstitiel, c'est-à-dire le fluide qui remplit l'espace entre les capillaires sanguins et les cellules. Il a en effet une composition ionique proche de celle du plasma sanguin.

Ces systèmes évolués permettent ainsi de surveiller les paramètres biochimiques souhaités de façon transcutanée, c'est-à-dire sans nécessité de percer régulièrement la peau et de prélever.

On connait des dispositifs avec microaiguilles, qui ont l'avantage d'être moins invasive que des aiguilles classiques. Toutefois, il est important que ces microaiguilles restent en place.

Il existe pour cela des dispositifs à demeure, où des microaiguilles sont maintenues sur la peau avec une bande adhésive. Toutefois, on souhaite pouvoir effectuer un contrôle en continu ou quasi-continu, ce qui requiert des dispositifs autonomes. On pourra citer le dispositif GlucoWatch, qui utilisait l'iontophorèse (et non pas des aiguilles). Ce principe est très douloureux (irritations fortes), si bien qu'il n'est pratiquement pas utilisable.

On connait aussi le dispositif du document WO2018104647, qui présente un boitier comprenant une capsule amovible, la capsule accueillant des microaiguilles configurées pour prélever du liquide interstitiel. Le boitier, quant à lui, accueille la majeure partie de l'électronique.

Ce dispositif portatif, typiquement au poignet, permet une mesure en continu et il suffit de changer la capsule pour changer de microaiguilles.

Toutefois, lorsqu'un tel dispositif est porté au poignet et qu'il est amovible, il génère des irritations cutanées qui sont d'autant plus forte que la personne est diabétique ou obèse. La difficulté réside dans l'acceptation par la peau de la présence récurrente de nombreuses microaiguilles, sans nécessairement devoir déplacer le dispositif d'un membre à l'autre.

L'invention vise à adresser ces difficultés.

### PRESENTATION DE L'INVENTION

Afin de résoudre certaines de ces difficultés, l'invention propose selon une premier aspect une capsule pour système de surveillance corporelle, la capsule comprenant :
- une face de couplage configurée pour être couplée avec une forme complémentaire d'un boitier,
- un réseau de microaiguilles positionnées sur une surface plane de contact et configurées pour être insérées dans la peau pour prélever ou analyser un fluide corporel du porteur du dispositif de surveillance corporelle ce dernier est positionné sur le membre,
caractérisé en ce que :
- la face de couplage présente une symétrie de rotation autour d'un axe de rotation orthogonal à la surface plane, autour duquel au moins deux positions de couplage sont autorisées avec une seule et unique forme complémentaire d'un boitier,
- aucune micro-aiguille n'est au même emplacement, dans un référentiel fixe, qu'une autre microaiguille dans les deux positions.

Dans un mode de réalisation, le réseau de microaiguilles occupe moins de la moitié de la surface de contact, les microaiguilles étant préférablement sur une même moitié de la surface de contact.

Dans un mode de réalisation, le réseau de micro-aiguilles occupe moins d'un tiers et trois positions au moins sont définies (ou un quart et quatre positions).

Dans un mode de réalisation, la face de couplage présente une forme inchangée pour le couplage avec un boitier selon les deux positions.

Dans un mode de réalisation, la capsule comprend sur la face de couplage un premier jeu de connecteurs électriques.

Dans un mode de réalisation, la capsule comprend sur la face de couplage un deuxième jeu de connecteurs électriques, redondant vis-à-vis du premier jeu de connecteurs électriques.

Dans un mode de réalisation, la capsule comprend un indicateur de position pour indiquer à l'utilisateur la position de la capsule.

Dans un mode de réalisation, l'indicateur de position est un indicateur visuel représenté sur la surface plane des microaiguilles, par exemple un mot/chiffre/signe ou deux mots/chiffres adjacents en position inversé l'un par rapport à l'autre.

Dans un mode de réalisation, l'indicateur de position est un indicateur électrique, par exemple à l'aide d'un contact électrique sur la face de couplage pour le repérage automatique par le boitier.

Selon le premier aspect, l'invention propose aussi un système de surveillance corporelle destiné à être attaché à un membre d'un être vivant, comprenant :
- un boitier, comprenant une face de couplage,
- une capsule telle que décrite précédemment,
la face de couplage du boitier présentant un unique emplacement pour recevoir la capsule dans les deux positions.

Dans un autre aspect, l'invention propose aussi un système de surveillance corporelle destiné à être attaché à un membre d'un être vivant, comprenant :
- un boitier, comprenant une face de couplage,
- une capsule comprenant des microaiguilles configurées pour être insérées dans la peau pour prélever et/ou analyser un fluide corporel du porteur du système de surveillance corporelle lorsque ce dernier est positionné sur le membre, la capsule comprenant une face de couplage,
le boitier et la capsule pouvant être couplées par le biais des faces de couplage,
dans lequel la capsule a deux positions différentes de couplage sur la face de couplage du boitier,
dans lesquelles les microaiguilles ne sont pas au même endroit entre les deux positions, dans le référentiel du boitier .

Ces deux aspects couvrent un même concept inventif commun, qui est celui d'un déplacement d'une capsule (rotation, translation par rapport à la capsule), mais avec des spécificités différentes : dans un cas, le déplacement de la capsule est transparent pour le boitier (ce qui signifie que les caractéristiques techniques et structurelles sont essentiellement la capsule); dans un autre cas, le boitier comprend plusieurs emplacements pour recevoir la capsule en différentes positions (ce qui signifie que les caractéristiques techniques et structurelles sont essentiellement la capsule).

Dans un mode de réalisation, dans le référentiel du boitier, le déplacement entre les deux positions est uniquement une rotation.

Dans un mode de réalisation, le référentiel du boitier, le déplacement entre les deux positions est uniquement une translation.

Pour tout système présenté précédemment, dans un mode de réalisation, le système comprend un boitier, le boitier comprenant une batterie et un processeur configuré pour recevoir les données relatives au liquide prélevé ou analysé par les microaiguilles.

Pour tout système présenté précédemment, dans un mode de réalisation, le boitier comprend deux ensembles redondants de connecteurs électriques, pour assurer la connexion électrique dans les deux positions de la capsule, en combinaison avec une capsule comprenant un seul jeu de connecteurs électriques.

Pour tout système présenté précédemment, dans un mode de réalisation, le système comprend un patch, la capsule étant attachable de façon amovible au patch et le patch étant attachable de façon amovible au boitier, le patch ayant un rôle d'adhésif.

Pour tout système présenté précédemment, dans un mode de réalisation, le patch comprend un orifice central à l'intérieur duquel se positionne la capsule, l'orifice ayant une forme complémentaire à la capsule, de sorte que la capsule puisse être positionnée dans le patch selon les deux positions.

Pour tout système présenté précédemment, dans un mode de réalisation, le système comprend un bracelet ou brassard ou lanière, configuré pour maintenir le boitier en place sur le membre.

Enfin, l'invention concerne un procédé de déplacement des microaiguilles à l'aide d'un système tel que défini précédemment, comprenant une étape de déplacement de capsule d'une première vers une deuxième position de sorte que les microaiguilles aient changé de position par rapport au boitier.

Le procédé peut comprendre une étape de retrait de la capsule puis une étape de remise en position de la capsule après avoir déplacer la capsule en rotation ou en translation,
Alternativement, le procédé peut comprendre une étape de coulissement de la capsule dans le boitier.

### PRESENTATION DES FIGURES

D'autres caractéristiques et avantages de la présente invention apparaîtront à la lecture de la description qui va suivre d'un mode de réalisation préférentiel. Cette description sera donnée en référence aux dessins annexés dans lesquels :
- La figure 1 illustre en vue éclatée un bracelet, un boitier, une capsule et un patch tel qu'utilisable dans le cadre de l'invention,
- Les figures 2a et 2b et 3 illustrent une vue tridimensionnelle d'une capsule : la face de contact avec microaiguille (deux vues) et la face de couplage,
- La figure 4 illustre l'agencement des différents modules qui forment le système (dimensions différentes des dimensions des figures 2a, 2b et 3),
- La figure 5 illustre un mode de réalisation de la face de couplage du boitier.

### DESCRIPTION DETAILLEE

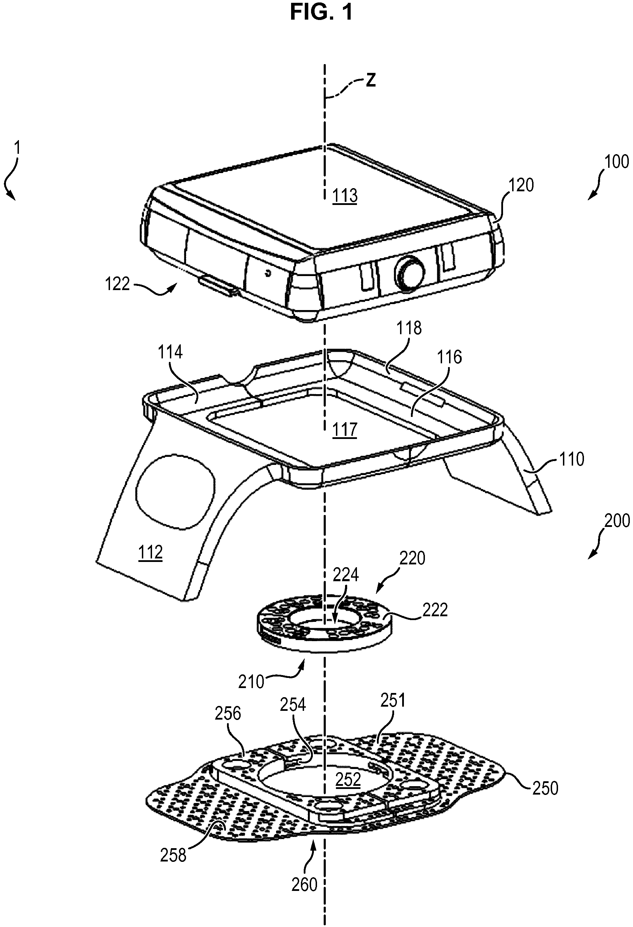

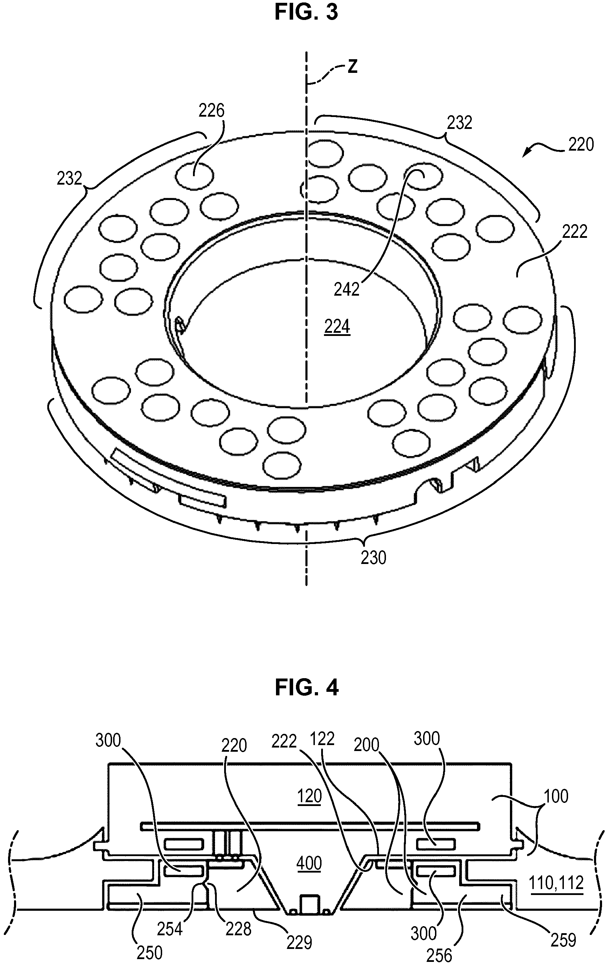

En référence aux **figures 1** **et** **4****,** la présente invention concerne un système électronique 1 de surveillance corporelle. Il s'agit d'une amélioration du dispositif du document WO2018104647. Par conséquent, l'invention se place dans le même concept général de système intégral autonome à faible douleur, faible risque hygiénique et réutilisable.

Par surveillance corporelle, on entend la vérification de constantes biochimiques d'une personne porteuse du système 1, typiquement la concentration en une protéine, une hormone, un marqueur, en oxygène, en nutriments, etc., dans le liquide interstitiel de la personne. On citera l'exemple de la glycémie. L'homme du métier pourra surveiller si besoin d'autres grandeurs physiques corporelles telles que le lactate, l'hydratation, etc.

La description sera illustrée avec du liquide interstitiel mais s'applique aux autres liquides corporels tels que le sang.

Le système 1 est dit autonome, car il ne nécessite pas l'utilisation de matériel supplémentaire.

Le système 1 est destiné à être attaché à un membre d'un être vivant, typiquement un bras ou une jambe d'un être humain. La zone d'attache privilégiée est le poignet où le système 1 s'apparente à une montre.

Le système 1 est formé de deux modules 100, 200 reliées entre eux par une liaison séparable 300. On définit ainsi une position couplée et une position libre. En position couplée, les deux modules 100, 200 ne sont pas séparés physiquement et peuvent échanger des données.

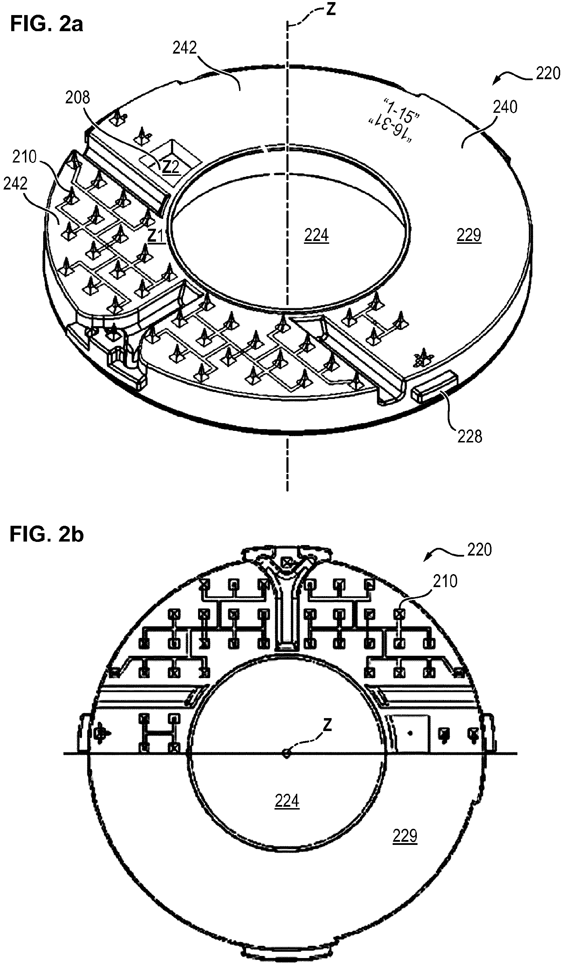

Le premier module 100 comprend un boitier 120 et préférablement des moyens d'attache et de serrage 100 du système 1 à un membre (de type lanière 112 ou bracelet). Le deuxième module 200 comprend une capsule 220 avec des microaiguilles de mesure 210 disposées sur une face de contact 229 et configurées pour être insérées dans la peau (dans une partie superficielle de l'épiderme). Ces microaiguilles de mesure 210, lorsque le premier module 100 est en position sur le membre, permettent de prélever et/ou d'analyser un fluide corporel, comme mentionné précédemment (voir **figures 2a et 2b**).

Les microaiguilles de mesure 210 consistent avantageusement en un réseau de microaiguilles de mesure 210 au contact de la peau lorsque la capsule 220 est placée sur le corps d'une personne. Les microaiguilles 210 peuvent donc être soit creuses, pour prélever du liquide, soit pleines, pour analyser directement le liquide. Dans le premier cas, typiquement, les microaiguilles 210 permettent l'extraction de liquide interstitiel du derme de façon indolore sans perlement de sang, et l'envoie vers un capteur. Dans le deuxième cas, les microaiguilles 210 ne prélèvent pas de fluide et intègrent le capteur sur leur surface, sous la forme d'un matériau biochimique apte à réagir avec l'analyte que l'on souhaite mesurer dans le fluide.

Il existe d'autres microaiguilles disposées sur la face de contact 229 (électrode de référence, etc.). On les référence aussi 210 comme les microaiguilles de mesure pour le reste de la description.

De façon préférée, les microaiguilles 210 comprennent entre quatre et cinquante microaiguilles 210, sensiblement pyramidales, avec des pointes d'une hauteur comprise entre 100µm et 1000µm, préférablement 0.3mm et 0.8mm. Chacune de ces caractéristiques avantageuses des microaiguilles 210 peut être prise séparément ou en combinaison avec les autres.

Comme expliqué précédemment, dans une variante, les microaiguilles 210 ne prélèvent pas de fluide et intègrent le capteur sur leur surface, sous la forme d'un matériau biochimique apte à réagir avec l'analyte que l'on souhaite mesurer dans le fluide. Dans une autre variante, les microaiguilles 210 prélèvent du fluide pour l'envoyer vers un capteur.

Les deux modules 100 et 200 présentent chacun une face de couplage 122, 222, de forme complémentaire, qui permet de placer le deuxième module 200 dans un emplacement d'accueil du premier module 100. On définit un plan de couplage relatif à la face de couplage 222 de la capsule 220. La face de couplage est donc mécanique.

Plusieurs modes de réalisation vont être présentés pour permettre de pouvoir déplacer les microaiguilles 210 sans déplacer le système de position sur le membre.

### Premier mode de réalisation : une seule et unique forme complémentaire du boitier 120

Dans ce premier mode de réalisation, la capsule 220 seule permet de résoudre les problèmes mentionnés en introduction.

Le boitier 120 présente, sur sa face de couplage 122, une seule et unique forme configurée pour recevoir la capsule 220. La face de couplage 122 de la capsule 220, quant à elle, présente une symétrie de rotation autour d'un axe Z orthogonal au plan de couplage. En d'autres termes, il existe au moins deux positions, obtenues successivement par rotation, pour lesquelles la forme face de couplage 222 de la capsule 200 demeure inchangée. Cette rotation peut être de 180° (comme illustré sur les **figures**) ou de 90° par exemple. La valeur de la rotation dépend de l'emplacement des microaiguilles 210 - cela sera expliqué par la suite.

De la sorte, la capsule 220 peut être couplée avec le boitier 120 selon au moins une première position et une deuxième position. Dans un référentiel fixe (par exemple le boitier 120), les deux positions diffèrent d'une rotation selon l'axe Z de la capsule 220.

En d'autres termes, la rotation de la capsule 220 est mécaniquement invisible pour le boitier 120, qui peut se coupler avec la capsule 220 en première et deuxième position dans la même forme complémentaire de sa face de couplage 122. En ce qui concerne la gestion du couplage électrique, si présent, il sera décrit par la suite.

Les microaiguilles 210 sont placées en réseau sur une surface de contact 229 de la capsule 220 de façon à ce que, dans un référentiel fixe (par exemple le boitier 120 ou le membre), aucune microaiguille 210 du réseau ne soit à un même emplacement qu'une microaiguille 210 du réseau entre la première position et la deuxième position.

A cette fin, lorsque la capsule 220 fonctionne selon deux positions, il faut que chaque microaiguille 210 n'ait pas d'équivalent sur la capsule 220 lorsqu'on applique la rotation (fictive) à la microaiguille. Une solution commode pour cela consiste à ne disposer le réseau de microaiguilles 210 que sur une seule moitié de la surface de contact 229. Par exemple, comme cela est illustré sur la **figure 2a et 2b****,** le réseau de microaiguilles 210 peut occuper une seule moitié au plus de la surface de contact (on entend ici la plus petite surface qui inclue toutes les microaiguilles 210 de la capsule 220 et non pas la somme de la surface de chaque microaiguilles 210). Il peut y avoir une légère tolérance à quelques microaiguilles près (2 ou 3 microaiguilles) ; préférablement, il n'y aucune tolérance. Après rotation à 180°, aucune microaiguille ne se trouvent au même emplacement, dans un référentiel fixe, qu'une microaiguille avant rotation. La peau est donc percée sur une nouvelle zone. En effet, quelques jours suffisent à recouvrer sa peau intacte. Or, la capsule doit être changée toutes les semaines environ (avec changement de position donc).

Dans l'exemple des **figures,** deux positions sont prévues (une seule rotation à 180°). Toutefois, il est possible de prévoir trois positions (deux rotations) : dans ce cas, le réseau de microaiguilles 210 n'est disposé que sur un tiers ou moins de la surface. Le principe est généralisable ; les seules contraintes étant l'espace disponible pour les microaiguilles 210 et les possibilités de couplage avec le boitier 120.

Il est aussi possible de prévoir une capsule 220 carrée avec deux positions (rotation à 90° ou 180° ou 270°) ou quatre positions (rotations à 90° entre deux positions successives).

En pratique, la face de couplage 122 du boitier 120 peut consister une surface essentiellement plane, avec éventuellement une saillie 400 qui fait office de guide, sur laquelle la capsule 220 peut être positionnée selon toute rotation. Toutefois, la capsule 220 est avantageusement attachée à un patch 250 (voir infra), qui lui est attaché au boitier 120. En conséquence, il s'agit du patch 250 qui détermine la valeur de rotation pour le positionnement sur la face de couplage 122 du boitier 120. De la même façon, la position de la capsule 220 dans le patch 250 est déterminée (l'utilisateur ne peut pas les agencer comme il le souhaite).

### Connecteurs électriques 226

Afin de pouvoir échanger des données avec le boitier 120, des jeux de connecteurs électriques 226 sont prévues sur la face de couplage 222 de la capsule 220 et sur la face de couplage 122 du boitier 120 (**figure 3**). Le jeu 230 de connecteurs 226 de la capsule 220 est relié au réseau de microaiguilles 210 (la plupart d'entre elles dans le cas de microaiguilles 210 qui mesurent ou seulement quelques unes d'entre elles dans le cas de microaiguilles 210 qui prélèvent) ou au capteur logé dans la capsule 220 (dans le cas de microaiguilles 210 qui prélèvent) pour pouvoir transmettre les informations obtenues par la capsule 220. Le jeu 230 de connecteurs du boitier 120 est typiquement relié notamment *in fine* à une batterie du boitier 120, des processeurs ou microprocesseurs, etc. Ces éléments du boitier 120 seront détaillés par la suite.

Les connecteurs électriques 226 sont par exemple réalisés sous forme de pins ou de pads métalliques, c'est-à-dire des pions métalliques. Lorsque deux connecteurs 226 sont mis en contact en vis-à-vis, une connexion électrique est assurée.

Du fait de la rotation de la capsule 220 entre deux positions, une redondance du jeu 230 de connecteurs 226 doit être prévue (**figure 3**).

Dans un mode de réalisation préférentiel, la redondance du jeu 230 de connecteurs 226 se trouve sur la face de couplage 222 de la capsule 220. Ainsi, pour deux positions, deux jeux de connecteurs 230, 232 redondants sont prévus : un premier 230 (qui contient une quinzaine de connecteurs 226), et un deuxième 232 (qui contient lui aussi une quinzaine de connecteurs 226), redondant du premier.

Par redondance, on entend que chaque jeu 230, 232 de connecteurs 226 peut remplir seul la fonction de connexion électrique de la capsule 220 au boitier 120 (pour un fonctionnement normal de la capsule évidemment) : il s'agit d'une forme de duplication.

Le positionnement des deux jeux 230, 232 est fait en dépendance du positionnement réseau de microaiguilles 210 et du jeu de connecteurs du boitier 120 pour s'assurer que lorsque la capsule 220 est en première et en deuxième position, ce soit bien deux jeux différents 230, 232 de connecteurs électriques 226 de la capsule 220 qui soient en contact avec le jeu de connecteurs complémentaires du boitier 120. Ainsi, dans l'exemple de la **figure 3****,** les deux jeux 230, 232 de connecteurs sont symétriques par la même rotation que celles mentionnées pour les microaiguilles 210 (le choix des numérotations 230, 232 est arbitraire sur les figures car les interconnexions électriques de redondance ne sont pas dessinées). Lorsque deux positions sont définies pour la microaiguille, chaque jeu 230, 232 de connecteurs occupe moins de la moitié de la surface de couplage 222.

Dans un autre mode de réalisation, la redondance des jeux de connecteurs se trouve sur la face de couplage 122 du boitier 120. Cela signifie qu'un seul jeu 230 de connecteurs 226 est nécessaire sur la surface de couplage 122 de la capsule 220. De la même façon, le positionnement des jeux de connecteurs redondants sur le boitier 120 dépend, lors de la conception, de la rotation prévue de la capsule 220.

### Connection sans fil

Alternativement aux connecteurs électriques 226, il est possible d'avoir un échange d'information entre la capsule 220 et le boitier 120 qui se fasse sans fil (*bluetooth,* infrarouge, RFID, NFC, etc.). Cela permet notamment de s'affranchir des problématiques liées à la redondance des connecteurs électriques, a *fortiori* quand on a une redondance avec au total trois ou quatre jeux de connecteurs (manque de place sur les faces de couplage). On peut alors prévoir une pile dans la capsule pour l'apport en énergie (conductimétrie, etc.).

### Indicateurs de position

Comme la capsule 220 se trouve sous le boitier 120 et n'ont pas forcément visible, il est capital que l'utilisateur puisse savoir dans quelle position est la capsule 220. De la même façon, une fois la capsule 220 retirée, l'utilisateur peut immédiatement oublier comment elle était positionnée ou bien se tromper de position entre le moment où il regarde la capsule 220 dans un sens puis la dépose sur la peau dans l'autre sens.

Pour résoudre ces difficultés, plusieurs indicateurs de position sont prévus.

Il peut y avoir un indicateur visuel 240, sur la surface de couplage 222 ou sur la face de contact 229, sous la forme d'un mot, d'un signe, d'un signe (flèche, etc.), avec éventuellement un signe complémentaire sur le boitier 120. Il peut aussi y voir deux mots ou chiffres adjacents en position inversé, de sorte qu'en fonction de la rotation à 180°, on sache dans quel sens est la capsule 220 (par exemple 1-15 et, juste dessous, à l'envers 16-31 pour savoir dans quelle position mettre la capsule 220 en fonction du jour du mois).

De la même façon, l'indicateur visuel peut être sur un patch aussi (voir infra).

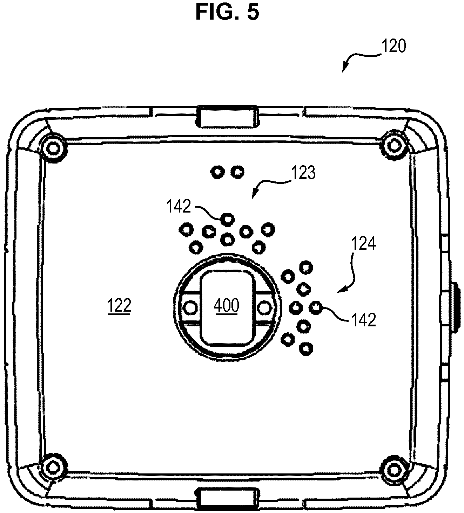

Il peut aussi y avoir un indicateur électrique 242 par exemple à l'aide d'un contact électrique additionnel 242 sur la face de couplage 222 (indicateur qui est aussi visuel de ce fait) qui est présent que dans un des jeux 230, 232 de connecteurs 226. En particulier, comme illustré sur la **figure 3****,** on remarque connecteur métallique supplémentaire qui vient rompre la symétrie des jeux 230, 232 de connecteurs 226. Lorsque ce connecteur 242 est en regard d'un connecteur électrique complémentaire 142 du boitier 120 (**figure 5**), un signal est détecté. Autrement, le connecteur électrique du boitier 120 est directement sur le plastique de capsule 220 et ne détecte aucun signal ou potentiel. Ainsi, le boitier 120 sait automatiquement dans quelle position est la capsule 220. Par conséquent, si la rotation est invisible pour le boitier 120 en matière de couplage mécanique, elle demeure repérable par le boitier 120 en matière de couplage électrique. Dans l'emplacement symétrique sans connecteur électrique additionnel 242, on peut y mettre l'indicateur visuel.

Le principe est le même si la redondance de jeu de connecteurs se trouve sur le boitier 120 : un connecteur additionnel est ajouté sur un des deux jeux.

### Deuxième mode de réalisation : plusieurs positions au sein du boitier 120

Dans ce mode de réalisation non illustré, la capsule 220 est mobile par rapport au boitier 120 selon au moins une translation ou rotation (voire les deux) entre une première position de couplage et une deuxième position de couplage. Le boitier 120 comprend donc une face de couplage 122 avec deux positions différentes pour la capsule 220 ou bien une face de couplage 122 mobile au sein du boitier 120.

Le mouvement est choisi afin qu'aucune des microaiguilles 210 ne soit à la même position qu'une autre micro-aiguille lorsque l'on compare l'emplacement des microaiguilles 210 des deux positions dans un référentiel fixe (boitier 120 ou poignet).

Dans une première variante, la capsule 220 se déplace par translation uniquement.

Des liaisons glissières peuvent convenir, avec une capsule 220 coulissante ou une bien une face de couplage 122 coulissante.

Dans une deuxième variante, la capsule 220 se déplace par rotation, par exemple sans que sa forme demeure inchangée par la rotation : en d'autres termes, le boitier 120 peut prévoir une face de couplage 122 avec deux formes complémentaires pour loger la capsule 220 dans les deux positions (au lieu d'une seule forme complémentaire comme dans le premier mode de réalisation).

### Compléments sur le système

La capsule 220 a une forme de boite fermée, typiquement étanche, qui peut se coupler avec le boitier 120. Cette capsule 220 est interchangeable, ce qui permet d'obtenir un système économique et efficace, où seules les parties dites consommables doivent être changées. La capsule 220 peut avoir une forme annulaire, avec une ouverture traversante 224 au centre. Dans une variante mentionnée précédemment, le capteur est positionné à l'intérieur de la capsule 220 (ou alors dans le boitier 120) et analyse le fluide prélevé par les microaiguilles 210.

De la même façon, le deuxième module 200 peut comprendre un patch 250, solidaire de façon amovible à la capsule 220. Le patch 250 fonctionne comme un adhésif pour maintenir les microaiguilles 210 enfoncées dans la peau. Le patch 250 est préférablement symétrique au niveau de l'ouverture, de façon à ce qu'il n'y ait pas de problème d'orientation du patch pour la mise en place sur le membre. Pour changer l'orientation de la capsule, l'utilisateur peut soit enlever patch+capsule et faire pivoter l'ensemble, soit enlever la capsule du patch, la faire pivoter et la remettre dans le patch, qui lui est réengagé avec le premier module 100 dans le même sens qu'originellement.

Des protubérances 228 sur une paroi externe de la capsule 220 sont configurés pour s'engager dans des encoches 254 du patch.

Comme mentionné précédemment, le premier module 100 comprend en outre un boitier 120 dans lequel sont disposés des moyens de traitement de données (en particulier un processeur ou un microcontrôleur) configurés pour traiter des mesures acquises par le capteur, et le cas échéant des moyens de stockage de données (notamment une mémoire, en particulier de type flash, et/ou la mémoire du microcontrôleur) permettant par exemple de stocker ces mesures, et/ou une date de la première utilisation de chaque capteur pour calculer une date de péremption du ou des capteurs (les capteurs biochimiques ont une durée de vie limitée). Les moyens de traitement de données servent aussi à générer des consignes vers différentes composants. Dans le cadre de cette description, ces différentes fonctions sont assurées par une même unité. Toutefois, il est possible de prévoir des processeurs dédiés. Le système comprend également une batterie pour l'alimentation électrique des composants, avantageusement rechargeable, par exemple via un port (dont on comprend qu'il peut également servir à connecter le système 1 par exemple à un ordinateur pour télécharger les données acquises et/ou traitées).

De façon préférée le système 1 peut comprendre des moyens de connexion sans fil (en particulier de type WiFi, mais également Bluetooth, voire 3G/4G) pour connexion à un réseau, en particulier internet, et une interface utilisateur tel qu'un écran, éventuellement tactile pour afficher les résultats de la surveillance à l'utilisateur.

L'homme du métier connait des algorithmes de traitement de mesures de capteurs et des interfaces associées, et saura les implémenter dans le présent système 1.

Comme indiqué précédemment, le boitier 120 comprend en outre des connecteurs électriques, sur sa face de couplage 122 avec la face de couplage 222 capsule 220.

Le deuxième module 200 forme un ensemble interchangeable du système qui est choisi selon le type de surveillance voulu et en fonction de l'état de détérioration des microaiguilles 210 et/ou du capteur.

En effet, dans la mesure où la capsule 220 contient les microaiguilles 210 et/ou le capteur, changer de capsule 220 permet de changer des éléments si ceux-ci sont en fin de vie ou si l'on souhaite changer de grandeur physique mesurée, en une manipulation simple, rapide et sure, sans devoir jeter d'autres parties (en particulier le premier module).

Et dans la mesure où la capsule 220 minimise la quantité d'élément et/ou matériaux coûteux (équipement électronique avancé tel qu'une batterie ou des moyens de communication sans fil), elle est relativement peu chère.

## Revendications

1. Capsule (220) pour système de surveillance corporelle (1), la capsule (220) comprenant :
- une face de couplage (222) configurée pour être couplée avec une forme complémentaire d'un boitier (120),
- un réseau de microaiguilles (210) positionnées sur une surface plane de contact (229) et configurées pour être insérées dans la peau pour prélever ou analyser un fluide corporel du porteur du dispositif de surveillance corporelle (1) lorsque ce dernier est positionné sur le membre,
**caractérisé en ce que** :
- la face de couplage (222) présente une symétrie de rotation autour d'un axe de rotation (Z) orthogonal à la surface plane (229), autour duquel au moins deux positions de couplage sont autorisées avec une seule et unique forme complémentaire d'un boitier (120),
- aucune micro-aiguille (210) n'est au même emplacement, dans un référentiel fixe, qu'une autre microaiguille (210) dans les deux positions.

2. Capsule (220) selon la revendication 1, dans lequel le réseau de microaiguilles (210) occupe moins de la moitié de la surface de contact (229), les microaiguilles (210) étant préférablement sur une même moitié de la surface de contact.

3. Capsule (220) selon la revendication 1 ou 2, dans laquelle la face de couplage (229) présente une forme inchangée pour le couplage avec un boitier (120) selon les deux positions.

4. Capsule (220) selon la revendication 1, comprenant sur la face de couplage (222) un premier jeu (230) de connecteurs électriques (226).

5. Capsule (220) selon la revendication 1, comprenant sur la face de couplage (222) un deuxième jeu (232) de connecteurs électriques, redondant vis-à-vis du premier jeu (230) de connecteurs électriques.

6. Capsule (220) selon la revendication 1, comprenant un indicateur de position (240, 242) pour indiquer à l'utilisateur la position de la capsule (220).

7. Capsule (220) selon la revendication 6, dans lequel l'indicateur de position est un indicateur visuel (240) représenté sur la surface plane des microaiguilles (210) ou sur la face de couplage (222), par exemple un mot / chiffre / signe ou deux mots/chiffres adjacents en position inversé l'un par rapport à l'autre.

8. Capsule (220) selon la revendication 6 ou 7 dans lequel l'indicateur de position est un indicateur électrique, par exemple à l'aide d'un contact électrique sur la face de couplage (222) pour le repérage automatique par le boitier (120).

9. Système de surveillance corporelle (1) destiné à être attaché à un membre d'un être vivant, comprenant :
- un boitier (120), comprenant une face de couplage (122),
- une capsule (220) selon l'une quelconque des revendications 1 à 8,
la face de couplage (122) du boitier (120) présentant un unique emplacement pour recevoir la capsule (220) dans les deux positions.

10. Système de surveillance corporelle (1) destiné à être attaché à un membre d'un être vivant, comprenant :
- un boitier (120), comprenant une face de couplage (122),
- une capsule (220) comprenant des microaiguilles (210) configurées pour être insérées dans la peau pour prélever et/ou analyser un fluide corporel du porteur du système de surveillance corporelle (1) lorsque ce dernier est positionné sur le membre, la capsule (220) comprenant une face de couplage (222),
le boitier (120) et la capsule (220) pouvant être couplées par le biais des faces de couplage (122, 222),
dans lequel la capsule (220) a deux positions différentes de couplage sur la face de couplage (122) du boitier,
dans lesquelles les microaiguilles (210) ne sont pas au même endroit entre les deux positions, dans le référentiel du boitier (120).

11. Système de surveillance corporelle (1) selon la revendication 10, dans lequel, dans le référentiel du boitier (120), le déplacement entre les deux positions est uniquement une rotation.

12. Système de surveillance corporelle (1) selon la revendication 10, dans le référentiel du boitier (120), le déplacement entre les deux positions est uniquement une translation.

13. Système de surveillance corporelle selon l'une quelconque des revendications 9 à 12, comprenant un boitier (120), le boitier (120) comprenant une batterie et un processeur configuré pour recevoir les données relatives au liquide prélevé ou analysé par les microaiguilles (210).

14. Système de surveillance corporelle selon la revendication 13, dans lequel le boitier (120) comprend deux ensembles redondants de connecteurs électriques, pour assurer la connexion électrique dans les deux positions de la capsule, en combinaison avec une capsule (220) comprenant un seul jeu de connecteurs électriques.

15. Système de surveillance corporelle (1) selon l'une quelconque des revendications 9 à 14 comprenant un patch (250), la capsule (220) étant attachable de façon amovible au patch (250) et le patch (250) étant attachable de façon amovible au boitier (120), le patch (250) ayant un rôle d'adhésif.
